# EUROPEAN PATENT SPECIFICATION

(11) **EP 0 696 183 B1**
(45) Date of publication and mention of the grant of the patent: **28.07.1999**
(21) Application number: 94914221.0
(22) Date of filing: 19.04.1994
(51) Int. Cl.: A61B 19/08

(54) **MODIFIED LITHOTOMY/PELVISCOPY SURGICAL DRAPE**
MODIFIZIERTES CHIRURGISCHES ABDECKTUCH FÜR LITHOTOMIE/PELVISKOPIE
DRAP CHIRURGICAL MODIFIE POUR LITHOTOMIE/PELVISCOPIE

(30) Priority: 29.04.1993 US 54997
(43) Date of publication of application: 14.02.1996
(73) Proprietor: KIMBERLY-CLARK WORLDWIDE, INC., Neenah, Wisconsin 54956 (US)
(72) Inventor: SIMPSON, Gwendolyn, Elizabeth, Riverdale, GA 30274 (US)
(74) Representative: Diehl, Hermann, Dr. Dipl.-Phys.
(86) International application number: US9404313
(87) International publication number: WO9424954

(56) References cited:
- EP-A- 0 167 506
- US-A- 2 593 121
- US-A- 3 856 006
- US-A- 3 910 268
- US-A- 4 196 723
- US-A- 4 471 769

## Description

The present invention relates to a surgical drape for a number of procedures including those when the patient is in the lithotomy or trendelenburg positions. More specifically, the present invention relates to a surgical drape with improved coverage of the operating room table and patient.

The number and type of surgical procedures are constantly on the rise. As new surgical procedures are developed, drape manufacturers will oftentimes design drapes which are more suitable for one or more type of procedures than another drape design. Many surgical procedures are performed using surgical drapes called "T-sheets". These sheets generally resemble the shape of a T and are constructed with a large sheet of material forming the top portion of the T horizontally attached to another sheet of material that is perpendicular to the larger sheet. Many of these sheets are sold as laparoscopy sheets and are used for surgical procedures from the perineal area to the abdominal area. These drapes will typically have one or two fenestrations with the lower fenestration adjacent the bottommost portion of the T, providing access to the perineal area while the upper fenestration provides access to the abdominal area. Another such drape with a similar configuration is a lithotomy drape. Both of these types of drapes are available from a number of manufacturers including Kimberly-Clark Corporation of Neenah, Wisconsin (assignee of record), Baxter Healthcare Corporation of Deerfield, Illinois, Johnson and Johnson Medical Incorporated of Arlington, Texas, and Boundary Healthcare Products Corporation of Columbus, Mississippi.

Surgical drapes are described in US-2,593,121, US-3,856,006, US-3,910,268 and US-4,196,723.

In connection with the surgical procedures using these drapes, it is common practice for the operating room personnel to move from the perineal area to the abdominal area of the patient during the surgical procedure. Typically during these procedures the patient is lying on the operating room table with his/her legs in the air in what are commonly referred to as the lithotomy and trendelenburg positions. In these positions, the long central portion of the "T-shaped" drape is positioned between the legs of the patient with the fenestration(s) covering the appropriate surgical site(s). The remainder of the drape, i.e. the upper horizontal portion of the drape forming the top of the T, is draped laterally across the upper portion of the patient. When operating room personnel move to the outside of the patient's legs to gain access to the patient's abdomen, the person is standing in an area where there is little or no coverage of the surgical table. One problem with the aforementioned drape design is that it does not provide total coverage and thus sterility of the operative site in this area of the patient/operating room table during the surgical procedure. There is therefore a need for a surgical drape providing such added coverage and protection.

One solution has been to provide a surgical drape of the aforementioned design with built-in leggings to cover the patient's legs while in an upright position. While this surgical drape design provides a greater sterile barrier than the preceding drape designs, the design is very difficult to unfold and lay out over the patient and operating room table.

### SUMMARY OF THE INVENTION

In view of the foregoing problems and disadvantages with previous surgical drapes, it is an object of the present invention to provide a surgical drape with side attachments or "wings" that will provide greater coverage of the patient and operating room table in the area to the side of and below the patient's legs.

The surgical drape of the present invention has two general embodiments. In the first embodiment, the surgical drape is made from a main sheet including a first portion and a second portion. The first portion of the surgical drape defines a cut-out section. The cut-out section is formed from at least two side edges, each of which extends inwardly from an edge of the first portion. The cut-out portion has a variety of shapes but the two edges will be generally angled inwardly so that the ends of the edges adjacent the edge of the first portion are closer together than the opposite ends of the two edges which terminate at a point in the interior of the first portion. The second portion of the main sheet of the surgical drape is connected to the first portion of the main sheet, usually at a point above the cut-out section in the first portion so that the second portion covers the cut-out section in the first portion of the main sheet and overlaps the two side edges of the cut-out portion. To provide access to the abdominal and perineal areas of the patient covered by the surgical drape, the main sheet can include a first fenestration located in the first portion and a second fenestration located in the second portion. If desired, a flap of material may be attached to the main sheet such that the flap can be pivoted to cover at least one of the first and second fenestrations.

In the second embodiment, the main sheet is generally "T-shaped" and includes a first portion corresponding to the upper portion of the "T" and a second portion corresponding to the lower portion of the "T". The first portion in this embodiment has a top edge, a left bottom edge and a right bottom edge with a pair of opposed side edges connecting the top edge respectively with the left bottom edge and the right bottom edge. The second portion includes a bottommost edge, a left side edge and a right side edge. Collectively these edges form the outline of the "T". Attached to the main sheet are a left flap portion and a right flap portion with the left flap portion extending and covering an area exterior to the left bottom edge and left side edge of the main sheet. The left flap portion includes a left overlap edge which overlaps the left side edge of the second portion of the main sheet. In the same fashion, the main sheet includes a right flap portion with a right overlap edge which extends and covers an area exterior of the right bottom edge and the right side edge with the right overlap edge overlapping the right side edge of the second portion of the main sheet. As with the other embodiment, the first portion may include a first fenestration and the second portion may include a second fenestration so as to provide access to the abdominal and perineal areas of the patient. In addition, a flap may be attached to the main sheet and pivotally adjusted to cover at least one of the first and second fenestrations. Lastly, the left overlap edge of the left flap portion and the right overlap edge of the right flap portion are generally angled inwardly.
Figure 1 is a top plan view of a modified lithotomy/pelviscopy surgical drape according to the present invention.
Figure 2 is a top plan view of another embodiment of a modified lithotomy/pelviscopy surgical drape according to the present invention.
Figure 3 is a perspective view of the surgical drape of Figure 1 positioned atop a patient.

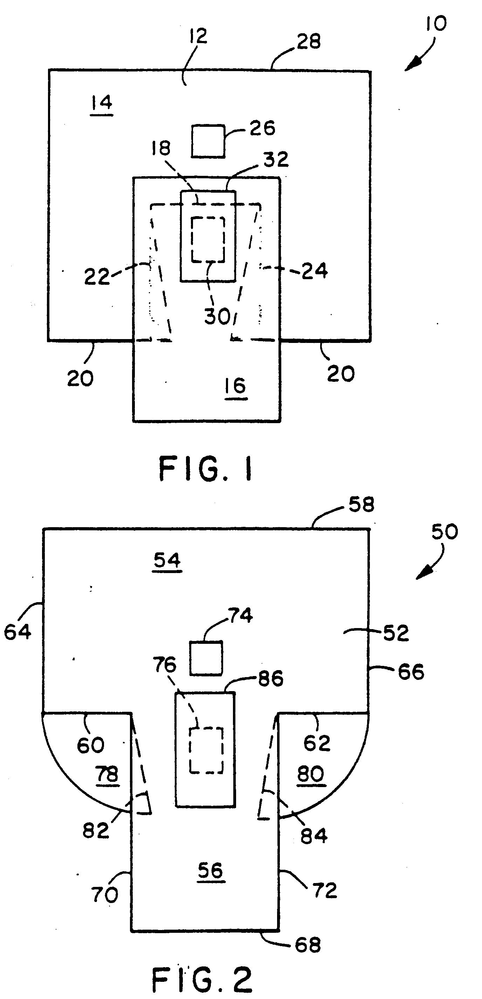

Referring to the drawings, there are at least two general drape designs corresponding to the present invention. The first, shown in Fig. 1, is based upon a "U-shaped" design with a second piece of material covering the center portion of the "U". The second design, shown in Fig. 2, more closely resembles a "T-shaped" design with wings attached to the "T" portion. For example, the drapes can be made from one or multiple pieces of material and still provide the same features and functions.

Referring specifically to Fig. 1, there is shown a surgical drape 10 made from a main sheet 12 including a first portion 14 and a second portion 16. The main sheet 12 including the first portion 14 and the second portion 16 can be made from any material or materials suitable for use in drape constructions. Examples of common draping materials include paper, plastic film, woven material such as linen, nonwoven materials, reinforced materials, foams, and laminates. Suitable nonwoven materials include spunbond, meltblown and staple fiber material such as bonded carded webs. One material particularly useful for the present invention and manufactured by the assignee of record, Kimberly-Clark Corporation, is a spunbond, meltblown, spunbond laminate sold under the trademark EVOLUTION® III fabric. This material can be made in accordance with the teachings of U.S. Patent No. 4,041,203 to Brock, et al., which is assigned to the assignee of record.

Turning again to Fig. 1, the first portion 14 of the main sheet 12 defines a cut-out section 18 extending inwardly from an edge 20 of the first portion 14. The cut-out section 18 is formed from at least two side edges 22 and 24 which extend inwardly from the edge 20 of the first portion 14. To perform surgical procedures within the abdominal area of the patient, the first portion 14 of the main sheet 12 may be provided with a first fenestration 26 which is usually located between the cut-out portion 18 and the top edge 28 of the first portion 14 of the main sheet 12. If desired, the fenestration 26 may be located in other areas of the first portion 14 of the main sheet 12.

The second portion 16 of the main sheet 12 shown in Fig. 1 is shown as having a generally rectangular shape. Other shapes are also contemplated to be within the scope of the present invention as defined by the appended claims provided the second portion 16 is of sufficient size to cover the cut-out section 18 in the first portion 14 of the main sheet 12. Generally, the second portion 16 will be attached or connected to the first portion 14 in the area of the first portion 14 between the cut-out section 18 and the top edge 28 and more particularly between the cut-out section 18 and the fenestration 26. The second portion 16 is of sufficient size to cover the cut-out portion 18 in the first portion 14 in a manner so as to overlap the two side edges 22 and 24 of the cut-out portion 18. By "overlap" it is meant that one piece of material lays on top of the other. In Figure 1, the two side edges 22 and 24 are underneath the second portion 16 but still overlap the second portion 16. It is usually desirable that the degree of overlap be at least 2.54 to 5.08 cm (about 1 to 2 inches) or greater so that during use, the overlap is still maintained. If desired, the second portion 16 may be provided with a second fenestration 30 to permit surgical procedures to the perineal area of the patient. Also if desired, the drape 10 may be provided with a flap 32 pivotally attached to the drape 10 such that it may be pivotally flipped back and forth to cover either the first fenestration 26 or the second fenestration 30.

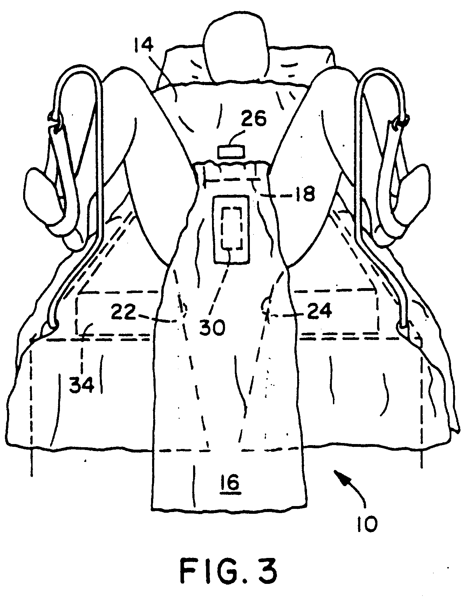

Referring to Fig. 3, the surgical drape 10 is shown positioned on an operating table 34 with the majority of the first portion 14 above the cut-out section 18 being positioned atop the table 34 with the remainder of the drape 10, including the second portion 16 extending down over the side of the table. In use, the patient lies underneath the drape 10 with the first fenestration 26 being positioned adjacent the abdominal region of the patient. The legs of the patient extend through the cut-out section 18 and are often placed in stirrups attached to the table 34. Next, the second portion 16 of the drape 10 is passed over the perineal area of the patient and the second portion is allowed to hang down over the side of the table in such a fashion so that the second portion 16 overlaps the two side edges 22 and 24 of the first portion 14 thereby providing a greater degree of coverage of the patient and the surgical table 34 than with previous surgical drape designs. As shown in Figs. 1 and 3, the two side edges 22 and 24 are angled inwardly toward one another to provide an added degree of overlap between these edges and the overlapping part of the second portion 16 of the surgical drape 10.

A second embodiment of a surgical drape according to the present invention is shown in Fig. 2. In this embodiment, the drape 50 includes a main sheet 52 including a first portion 54 and a second portion 56. As can be seen, the first and second portions 54 and 56 form a generally "T-shaped" design with the first portion 54 forming the cross-bar of the "T" and the second portion 56 forming the main stem of the "T". As with the design shown in Figs. 1 and 3, this design may be made from the same types of materials as described above and also may be made from one or more pieces of material.

Referring to Fig. 2, the first portion 54 of the main sheet 52 includes a top edge 58, a left bottom edge 60, a right bottom edge 62, and a pair of opposed side edges 64 and 66. The second portion 56 includes a bottom-most edge 68, a left-side edge 70, and a right-side edge 72. As shown in Fig. 2, the first portion 54 and second portion 56 of main sheet 52 are formed from one piece of material. To permit access to the abdominal region of a patient (not shown) the first portion 54 defines a first fenestration 74 located between the top edge 58 and the left and right bottom edges 60 and 62. To permit access to the perineal area of the patient (not shown) the second portion 56 may define a second fenestration 76 located between the first portion 54 and the bottom-most edge 68.

To provide increased coverage of the patient and operating table, the drape 50 in Fig. 2 includes a left-flap portion 78 and a right-flap portion 80. The left-flap portion 70 either extends from or is attached to the first portion 54 adjacent or at the left-bottom edge 60 and includes a left-overlap edge 82 designed to overlap all or a portion of the left-side edge 70 of the second portion 56 of the drape 50. Generally, the left-flap portion 78 will extend and cover an area exterior to the left-bottom edge 60 and the left-side edge 70. In the same fashion, the right-flap portion 80 performs the same function as the left-flap portion 78 but on the opposite side of the second portion 56 of the drape 50. Consequently, the right-flap portion 80 extends and covers an area exterior to the right-bottom edge 62 and the right-side edge 72 with the right-flap portion 80 overlapping all or a portion of the right-side edge 72 of the second portion 56. As with the design shown in Figs. 1 and 3, it is generally desirable to have at least a 2.54 to 5.08 cm (1 to 2 inch) overlap between each of the flap portions and the side edges of the second portion of the main sheet 52. As with the design shown in Figs. 1 and 3, the design in Fig. 2 has left and right overlap edges 82 and 84 which are angled inwardly as specifically drawn in Fig. 2. In fact, the degree of angling of the left and right overlap edges 82 and 84 can be sufficiently severe so as to cause the two edges to overlap one another (not shown) and even be attached to one another if so desired so as to further maximize the degree of isolation of the patient and surgical table (not shown) from the sterile field of operation.

In the same fashion as the embodiment shown in Figs. 1 and 3, the second portion 56 may be provided with a second fenestration 76 and a flap 86 may be pivotally attached to the drape 50 such that it may be pivotally flipped back and forth to cover either the first fenestration 74 or the second fenestration 76.

Use of the surgical drape 50 shown in Fig. 2 is identical to the use described with respect to the surgical drape 10 shown in Figs. 1 and 3 with the advantage being the added degree of isolation of the patient and operating room table due to the use of the wings or flaps 78 or 80 which overlap the second portion 56 of the drape 50.

## Claims

1. A surgical drape (10) comprising:
a main sheet (12) including a first portion (14) and a second portion (16),
said first portion (14) defining, a cut-out section (18) extending inwardly from an edge (20) of said first portion (14), laid cut-out section (18) being formed from two side edges (22,24) extending inwardly into said first portion (14) from said edge (20) of said first portion (14),
said second portion (16) being connected to said first portion (14), said second portion (16) covering said cut-out section (18) in said first portion (14) and overlapping said two side edges (22,24),
characterized in that
said two side edges (22, 24) are angled inwardly toward one another, wherein each of said side edges (22, 24) and said edge (20) of the first portion (14) form an angle of less than 90°.

2. The surgical drape (10) according to claim 1 wherein said second portion (16) is connected to said first portion (14) between said cut-out section (18) and said top edge (28) of said first portion (14).

3. A surgical drape (50) comprising:
a generally "T-shaped" main sheet (52) including a first portion (54) corresponding to the upper portion of said "T" and a second portion (56) corresponding to the lower portion of said "T", said first portion (54) having a top edge (58), a left bottom edge (60) and a right bottom edge (62) and a pair of opposed side edges (64,66), said second portion (56) having a bottommost edge (68), a left side edge (70) and a right side edge (72),
said main sheet (52) further including a left flap portion (78) and a right flap portion (80), said left flap portion (78) extending and covering an area exterior of said left bottom edge (60) and said left side edge (70), said left flap portion (78) overlapping said left side edge (70), said right flap portion (80) extending and covering an area exterior of said right bottom edge (62) and said right side edge (72), said right flap portion (80) overlapping said right side edge (72),
characterized in that
said left flap portion (78) has a left overlap edge (82) which overlaps said left side edge (70) of said second portion (56) and said right flap portion (80) has a right overlap edge (84) which overlaps said right side edge (72) of said second portion (56), said left and right overlap edges (82, 84) being angled inwardly toward one another, wherein said left overlap edge (82) and said left bottom edge (60) as well as said right overlap edge (84) and said right bottom edge (62) respectively form an angle of more than 90°.

4. The surgical drape of claim 3 wherein said left and said right overlap edges (82, 84) overlap one another.

5. The surgical drape (10;50) of any of the preceding claims wherein said first portion (14;54) of said main sheet (12;52) defines a first fenestration (26;74) therein and said second portion (16,56) of said main sheet (12;52) defines a second fenestration (30;76) therein.

6. The surgical drape of any of the preceding claims wherein said drape (10;50) further includes a flap (32;86) attached to said main sheet (12;52), said flap (32;86) being capable of covering at least one of said first (26;74) and second fenestrations (30;76).

## Patentansprüche

1. Chirurgischer Behang (10) mit
einem Haupttuch (12) mit einem ersten Bereich (14) und einem zweiten Bereich (16),
wobei der erste Bereich (14) einen ausgeschnittenen Abschnitt (18) ausbildet, welcher sich einwärts von einer Kante (20) des ersten Bereichs (14) erstreckt, wobei der ausgeschnittene Bereich (18) von zwei Seitenkanten (22, 24) gebildet ist, welche sich einwärts in den ersten Bereich (14) von der Kante (20) des ersten Bereichs (14) erstrecken,
wobei der zweite Bereich (16) mit dem ersten Bereich (14) verbunden ist, wobei der zweite Bereich (16) den ausgeschnittenen Bereich (18) in dem ersten Bereich (14) bedeckt und die beiden Seitenkanten (22, 24) überlappt,
dadurch gekennzeichnet, daß
die beiden Seitenkanten (22, 24) einwärts zueinander winkelig sind, wobei jede der Seitenkanten (22, 24) und die Kante (20) des ersten Bereichs (14) einen Winkel von weniger als 90° bilden.

2. Chirurgischer Behang (10) gemäß Anspruch 1, bei dem der zweite Bereich (16) mit dem ersten Bereich (14) zwischen dem ausgeschnittenen Bereich (18) und der oberen Kante (28) des ersten Bereichs (14) verbunden ist.

3. Chirurgischer Behang (50) mit:
einem im allgemeinen "T-förmigen" Haupttuch (52) mit einem ersten Bereich (54) entsprechend dem oberen Bereich des "Ts" und einem zweiten Bereich (56) entsprechend dem unteren Bereich des "Ts", wobei der erste Bereich (54) eine obere Kante (58), eine linke untere Kante (60) und eine rechte untere Kante (62) und ein Paar entgegengesetzter Seitenkanten (64, 66) aufweist, wobei der zweite Bereich (56) eine unterste Kante (68), eine linke Seitenkante (70) und eine rechte Seitenkante (72) aufweist, wobei das Haupttuch (52) des weiteren einen linken Klappenbereich (78) und einen rechten Klappenbereich (80) aufweist, wobei der linke Klappenbereich (78) einen Bereich außerhalb der linken unteren Kante (60) und der linken Seitenkante (70) verläuft und bedeckt, wobei der linke Klappenbereich (78) die linke Seitenkante (70) überlappt, wobei, der rechte Klappenbereich (80) einen Bereich außerhalb der rechten unteren Kante (62) und der rechten Seitenkante (72) verläuft und bedeckt, wobei der rechte Klappenbereich (80) die rechte Seitenkante (72) überlappt,
dadurch gekennzeichnet, daß
der linke Klappenbereich (78) eine linke Überlappungskante (82) aufweist, welche die linke Seitenkante (70) des zweiten Bereichs (56) überlappt und der rechte Klappenbereich (80) eine rechte Überlappungskante (84) aufweist, welche die rechte Seitenkante (72) des zweiten Bereichs (56) überlappt, wobei die rechte und die linke Überlappungskante (82, 84) einwärts zueinander winkelig sind, wobei die linke Überlappungskante (82) und die linke untere Kante (60) sowie die rechte Überlappungskante (84) und die rechte untere Kante (62) jeweils einen Winkel von mehr als 90° bilden.

4. Chirurgischer Behang gemäß Anspruch 3, bei dem die linke und die rechte Überlappungskante (82, 84) einander überlappen.

5. Chirurgischer Behang (10; 50) gemäß einem der vorhergehenden Ansprüche, bei dem der erste Bereich (14; 54) des Haupttuches (12; 52) eine erste Fensterung (26; 74) hierin aufweist und der zweite Bereich (16; 56) des Haupttuches (12; 52) eine zweite Fensterung (30; 76) hierin aufweist.

6. Chirurgischer Behang gemäß einem der vorhergehenden Ansprüche, wobei der Behang (10; 50) des weiteren eine an dem Haupttuch (12; 52) angebrachte Klappe (32; 86) aufweist, wobei die Klappe (32; 86) zumindest eine Fensterung ausgewählt aus der ersten (26; 74) und der zweiten Fensterung (30; 76) bedecken kann.

## Revendications

1. Champ chirurgical (10) formé :
d'une feuille principale (12) comprenant une première portion (14) et une seconde portion (16),
ladite première portion (14) définissant une section découpée (18) s'étendant vers l'intérieur depuis un bord (20) de ladite première portion (14), ladite section découpée (18) étant formée depuis deux bords latéraux (22,24) s'étendant vers l'intérieur jusque dans ladite première portion (14) depuis ledit bord (20) de ladite première portion (14),
ladite seconde portion (16) étant connectée à ladite première portion (14), ladite seconde portion (16) couvrant ladite section découpée (18) définie dans ladite première portion (14) et chevauchant lesdits deux bords latéraux (22,24),
caractérisé en ce que
lesdits deux bords latéraux (22,24) sont en biais vers l'intérieur en direction l'un de l'autre, chacun desdits bords latéraux (22,24) formant avec ledit bord (20) de la première portion (14) un angle inférieur à 90°.

2. Champ chirurgical (10) selon la revendication 1, dans lequel ladite seconde portion (16) est connectée à ladite première portion (14) entre ladite section découpée (18) et le bord supérieur (28) de ladite première portion (14).

3. Champ chirurgical (50) comprenant :
une feuille principale (52) généralement en forme de "T" comprenant une première portion (54) correspondant à la partie supérieure dudit "T" et une seconde portion (56) correspondant à la partie inférieure dudit "T", ladite première portion (54) ayant un bord supérieur (58), un bord inférieur gauche (60), un bord inférieur droit (62) et une paire de bords latéraux opposés (64,66), ladite seconde portion (56) ayant un bord inférieur extrême (68), un bord latéral gauche (70) et un bord latéral droit (72),
ladite feuille principale (52) comprenant, en outre, une portion de volet gauche (78) et une portion de volet droit (80), ladite portion de volet gauche (78) s'étendant depuis ledit bord inférieur gauche (60)et couvrant une aire extérieure audit bord inférieur gauche (60) et audit bord latéral gauche (70), ladite portion de volet gauche (78) chevauchant ledit bord latéral gauche (70), ladite portion de volet droit (80) s'étendant depuis ledit bord inférieur droit (62)et couvrant une aire extérieure audit bord inférieur droit (62) et audit bord latéral droit (72), ladite portion de volet droit (80) chevauchant ledit bord latéral droit (72),
caractérisé en ce
ladite portion de volet gauche (78) a un bord de chevauchement gauche (82) qui chevauche ledit bord latéral gauche (70) de ladite seconde portion (56) et ladite portion de volet droit (80) a un bord de chevauchement droit (84) qui chevauche ledit bord latéral droit (72) de ladite seconde portion (56), lesdits bords de chevauchement gauche et droit (82,84) étant en biais vers l'intérieur en direction l'un de l'autre, ledit bord de chevauchement gauche (82) et ledit bord de chevauchement droit (84) formant respectivement avec ledit bord inférieur gauche (60) et ledit bord inférieur droit (62) un angle supérieur à 90°.

4. Champ chirurgical selon la revendication 3, dans lequel lesdits bords de chevauchement gauche et droit (82,84) se chevauchent l'un l'autre.

5. Champ chirurgical (10;50) selon l'une quelconque des revendications précédentes, dans lequel ladite première portion (14;54) de ladite feuille principale (12;52) définit une première fenêtre (26;74) dans ladite feuille principale et dans lequel ladite seconde portion (16;56) de ladite feuille principale (12;52) définit une seconde fenêtre (30;76) dans ladite feuille principale.

6. Champ chirurgical selon l'une quelconque des revendications précédentes, dans lequel ledit champ (10;50) comprend en outre un volet (32;86) fixé à ladite feuille principale (12;52), ledit volet (32;86) étant capable de couvrir au moins l'une desdites première (26;74) et seconde (30;76) fenêtres.
